# EUROPÄISCHE PATENTSCHRIFT

(11) **EP 3 583 923 B1**
(45) Veröffentlichungstag und Bekanntmachung des Hinweises auf die Patenterteilung: **28.06.2023**
(21) Anmeldenummer: 19179360.3
(22) Anmeldetag: 15.04.2016
(51) Int. Cl.: A61F 2/50

(54) **VERFAHREN ZUR STEUERUNG EINES KÜNSTLICHEN KNIEGELENKS**
METHOD OF CONTROLLING AN ARTIFICIAL KNEE JOINT
PROCÉDÉ DE COMMANDE D'UNE ARTICULATION ARTIFICIELLE DU GENOU

(30) Priorität: 24.04.2015 DE 102015106391
(43) Veröffentlichungstag der Anmeldung: 25.12.2019
(62) Teilanmeldung aus: 16716572.9
(73) Patentinhaber: Otto Bock Healthcare Products GmbH, 1110 Wien (AT)
(72) Erfinder: Seifert, Dirk, 1070 Wien (AT)
(74) Vertreter: Gramm, Lins & Partner Patent- und Rechtsanwälte PartGmbB

(56) Entgegenhaltungen:
- EP-A1- 2 278 942
- WO-A1-2009/141115
- US-A1- 2010 228 360
- US-A1- 2012 226 364

## Beschreibung

Die Erfindung betrifft ein Verfahren zur Steuerung eines künstlichen Kniegelenkes, insbesondere die Steuerung einer Dämpfungsveränderung eines künstlichen Kniegelenkes, das ein Oberteil, ein um eine Schwenkachse schwenkbar daran gelagertes Unterteil und eine Widerstandseinheit aufweist, wobei die Widerstandseinheit zwischen dem Oberteil und dem Unterteil an einem oberen Anlenkpunkt an dem Oberteil und an einem unteren Anlenkpunkt an dem Unterteil gelagert ist und einen Flexionswiderstand gegen das Einbeugen bereitstellt, wobei der Widerstandseinheit eine Verstelleinrichtung zur Veränderung des Flexionswiderstandes zugeordnet ist.

Kniegelenke für Orthesen, Exoskelette oder Prothesen weisen ein Oberteil mit einem oberen Anschlussteil und einem Unterteil mit einem unteren Anschlussteil auf, die gelenkig miteinander verbunden sind. An dem oberen Anschlussteil sind in der Regel Aufnahmen für einen Oberschenkelstumpf oder eine Oberschenkelschiene angeordnet, während an dem unteren Anschlussteil ein Unterschenkelrohr oder eine Unterschenkelschiene angeordnet sind. Im einfachsten Fall sind das Oberteil und das Unterteil durch ein einachsiges Gelenk verschwenkbar miteinander verbunden.

Um unterschiedliche Anforderungen während der verschiedenen Phasen eines Schrittes oder bei anderen Bewegungen oder Verrichtungen möglichst natürlich erfüllen zu können oder zu unterstützen, ist häufig eine Widerstandseinrichtung vorgesehen, die einen Flexionswiderstand und einen Extensionswiderstand bereitstellt. Über den Flexionswiderstand wird eingestellt, wie leicht sich das Unterteil im Verhältnis zum Oberteil bei einer aufgebrachten Kraft nach hinten schwingen lässt. Der Extensionswiderstand bremst die Vorwärtsbewegung des Unterteils und bildet unter anderem einen Streckanschlag aus.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssenor, ein Neigungssenor und/oder ein Kraft-sensor. In Abhängigkeit von den Sensordaten wird die Position des Extensionsanschlages ermittelt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2009 052 887 A1 beschreibt unter anderem ein Verfahren zur Steuerung eines orthetischen oder prothetischen Gelenkes mit einer Widerstandseinrichtung und Sensoren, wobei über Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beuge- und/oder Streckwiderstandes zugrunde gelegt wird.

Zur Steuerung der Veränderung des Dämpfungsverhaltens werden gemäß dem Stand der Technik die Sensordaten quantitativ ausgewertet, das heißt, dass in der Regel bestimmte Grenzwerte vorgegeben werden, bei deren Erreichen oder Nichterreichen der Aktuator aktiviert oder deaktiviert wird, so dass die Widerstandseinrichtung einen erhöhten oder verringerten Flexions- oder Extensionswiderstand bereitstellt.

Aus der DE 10 2008 024 747 A1 und der DE 103 51 916 A1 sind weitere prothetische Kniegelenke bekannt, mit denen ein Hinsetzen möglich ist. Ein Steuerungsverfahren gemäss der Präambel von Anspruch 1 ist aus WO 2009/141115 bekannt.

Widerstandseinheiten mit Anlenkpunkten oder Lagerungsstellen an dem Oberteil und dem Unterteil werden notwendigerweise in einem Abstand von der Schwenkachse, in der Regel in Gehrichtung hinter der Schwenkachse, montiert. Die Wirkungslinie der auf die Widerstandseinheit wirkenden Kraft zwischen dem oberen Anlenkpunkt und dem unteren Anlenkpunkt verläuft somit beabstandet zu der Schwenkachse, um die Rotationsbewegung in eine Translationsbewegung der Widerstandseinheit umwandeln zu können. Rotationshy-drauliken weisen keine Anlenkpunkte an dem Oberteil bzw. Unterteil auf.

Der Abstand der innerhalb der Widerstandseinheit zwischen den Anlenkpunkten verlaufenden Kraftwirkungslinie zu der Schwenkachse ist abhängig von dem Kniewinkel. Als Abstand wird die Länge einer Senkrechten auf die Wirkungslinie, die durch die Schwenkachse verläuft, angesehen. Der Abstand definiert gleichzeitig einen Hebelarm, der abhängig von dem Kniewinkel ist.

Vielfach fällt es Patienten schwer, das Körpergewicht bei der Bewegung vom Stehen zum Sitzen mit dem unversorgten Bein aufzufangen, so dass häufig die Hände zur Abstützung hinzugenommen werden müssen.

Aufgabe der vorliegenden Erfindung ist es, ein Verfahren bereitzustellen, mit dem einem Patient das sich Hinsetzen erleichtert wird.

Erfindungsgemäß wird diese Aufgabe durch ein Verfahren mit den Merkmalen des Hauptanspruches gelöst, vorteilhafte Ausgestaltungen und Weiterbildungen der Erfindung sind in den Unteransprüchen, der Beschreibung sowie den Figuren gezeigt.

Das Verfahren zur Steuerung eines künstlichen Kniegelenkes, insbesondere einer Dämpfungsveränderung an einem künstlichen Kniegelenk, das ein Oberteil, ein um eine Schwenkachse schwenkbar daran gelagertes Unterteil und einer zwischen dem Oberteil und dem Unterteil angeordneten Widerstandseinheit aufweist, wobei die Widerstandseinheit an einem oberen Anlenkpunkt an dem Oberteil und an einem unteren Anlenkpunkt an dem Unterteil gelagert ist und einen Flexionswiderstand gegen das Einbeugen bereitstellt, wobei der Widerstandseinheit eine Verstelleinrichtung zur Veränderung des Flexionswiderstandes zugeordnet ist, sieht vor, dass der Widerstand der Widerstandseinheit ab einem Kniewinkelschwellwert mit zunehmendem Kniewinkel vergrößert wird. Aufgrund der Abhängigkeit des Widerstandsmomentes von dem Hebelarm, der sich durch den Abstand der Kraftwirkungslinie der Widerstandseinheit zu der Schwenkachse ergibt, besteht das Problem, dass bei einem zunehmenden Kniewinkel ab Erreichen einer Maximallänge des Hebelarmes das Widerstandsmoment gegen eine Flexion, das Flexionsmoment abnimmt, wenn der Dämpfungswiderstand innerhalb der Widerstandseinheit konstant bleibt. Dies ergibt sich aus den geometrischen Beziehungen zwischen dem Oberteil, dem Unterteil, den jeweiligen Anlenkpunkten und der Kniewinkelstellung. Gerade im Bereich einer Knieeinbeugung von über 30° verringert sich üblicherweise die Hebelarmlänge, so dass erfindungsgemäß vorgesehen ist, dass der Flexionswiderstand der Widerstandseinheit mit zunehmendem Flexionswinkel vergrößert wird, um einen Abfall des Widerstandsmomentes gegen ein Einbeugen zu verringern, zu kompensieren oder eine Überkompensation bereitzustellen. Wird also eine Hinsetzbewegung erkannt, wird ab Erreichen eines festgelegten Schwellwinkels die Widerstandseinheit so durch die Verstelleinrichtung verändert, dass sich ein vergrößernder Widerstand ergibt. Dadurch ist es möglich, die sich verringernde Hebellänge, also der sich verringernde Abstand der Wirkungslinie innerhalb der Widerstandseinheit auszugleichen und in dem Bereich des Kniewinkels, in dem beim Hinsetzen ein vergrößertes Widerstandsmoment benötigt wird, eine Erhöhung des Widerstandes in der Widerstandseinheit und damit eine Erhöhung des Widerstandsmomentes verglichen mit einem unveränderten Widerstand der Widerstandseinheit bereitzustellen. Das Verfahren ist zur Steuerung sowohl von Prothesen als auch von Orthesen und Exoskeletten vorgesehen. Wird nachfolgend von Orthesen gesprochen, so gelten die Ausführungen ebenso für die Sonderform der Orthese in Gestalt eines Exoskelettes.

Der Widerstand innerhalb der Widerstandseinheit kann linear, progressiv oder degressiv erhöht werden. Der Grad der Erhöhung ebenso wie der Verlauf der Widerstandserhöhung hängt von dem zu erreichenden Widerstandsmoment ab. Ebenfalls hängt der Verlauf und der Grad der Widerstandserhöhung davon ab, wie sich die Hebellänge über den Kniewinkel verändert und ob eine Abmilderung der Verringerung des Widerstandsmomentes, eine Beibehaltung des eingestellten Widerstandsmomentes oder eine Überkompensation der Verringerung des Widerstandsmomentes erfolgen soll, also ob das Widerstandsmoment mit zunehmendem Kniewinkel vergrö-ßert werden soll. Das Widerstandsmoment ergibt sich aus dem Produkt des Dämpfungswiderstandes mit der Hebellänge, also des Abstandes der Kraftwirkungslinie der Widerstandseinheit zu der Schwenkachse. Die Veränderung des Widerstandes innerhalb der Widerstandseinheit wird beim sich Niedersetzen gesteuert, wodurch insgesamt das Widerstandsmoment und damit die Dämpfung erhöht wird.

Als Kniewinkelschwellwert, ab dem der Widerstand der Widerstandseinheit erhöht wird, kann derjenige Kniewinkel festgelegt werden, bei dem der Abstand der Verbindungslinie des oberen Anlenkpunktes zum unteren Anlenkpunkt zu der Schwenkachse maximal ist.

Die Widerstandseinheit kann einen hydraulischen oder pneumatischen Dämpfer mit zumindest einem Überströmkanal und einer verstellbaren Drossel aufweisen. Die Veränderung des Widerstandes innerhalb der Widerstandseinheit wird dann durch eine Verringerung des Strömungsquerschnittes innerhalb der Drossel durch die Verstelleinrichtung bewirkt.

Die Widerstandseinheit kann ebenfalls einen mechanischen Widerstand aufweisen, bei dem über einen Reibungswiderstand das Widerstandsmoment gegen die Einbeugung aufgebracht wird. Beispielsweise kann durch eine Erhöhung des Anpressdruckes in dem mechanischen Widerstand der Widerstand der Widerstandseinheit und damit auch das Flexionsmoment erhöht werden.

Die Widerstandseinheit kann als elektrisch betriebener Aktuator ausgebildet sein, die Veränderung des Widerstandes der Widerstandseinheit kann durch Erhöhung des elektrischen Widerstandes innerhalb des Aktuators individuell eingestellt werden.

Vorteilhafterweise wird der Widerstand in der Widerstandseinheit so erhöht, dass das Flexionsmoment in einem Kniewinkelbereich zwischen 70° und 90° maximal ist. Vorteilhafterweise wird das Flexionsmoment ab einem Kniewinkelschwellwert von 30° durch Vergrößerung des Widerstandes der Widerstandseinheit erhöht. Der Kniewinkel wird ausgehend von einer Streckstellung mit zunehmender Einbeugung als zunehmender Kniewinkel gemessen, in der Streckstellung beträgt der Kniewinkel somit 0°.

Eine Weiterbildung der Erfindung sieht vor, dass der Widerstand in der Widerstandseinheit umgekehrt proportional zum Verlauf der Hebelarmveränderung während des Einbeugens verändert wird, um ein konstantes oder nahezu konstantes Flexionsmoment bereitzustellen. Der Widerstand der Widerstandseinheit und damit auch das Flexionsmoment werden so erhöht, dass das Widerstandsmoment um die Knieachse konstant oder nahezu konstant gehalten wird, so dass der Patient keine Veränderung in dem Verhalten der Orthese oder Prothese spürt. Verändert sich der wirksame Hebelarm beispielsweise linear und verringert sich über den sich verringernden Kniewinkel, wird der Widerstand ebenfalls linear erhöht, so dass das Produkt aus Hebelarm und Widerstand über den gesamten Kniewinkelbereich konstant oder nahezu konstant bleibt. Gleiches gilt für degressive oder progressive Veränderungen im Hebelarm über den Kniewinkelverlauf entsprechend, es sind dann progressive oder degressive Veränderungen des Widerstandes vorgesehen, die mit der Veränderung des Hebelarmes korrelieren. Die Widerstandsänderungen können über Kurvenscheiben, andere mechanische Verstelleinrichtungen oder über elektronische Verstellprogramme ausgeführt werden. Da sich die Charakteristik der Hebelarmveränderung nicht ändert und der Hebelarm nur von dem Kniewinkel abhängig ist, kann die Anpassung der Widerstandsänderung einmalig festgelegt werden.

Der Anfangswiderstand wird auf einem Niveau eingestellt, das einem Standphasendämpfungsniveau entspricht, es wird also mit einem hohen Widerstand gestartet, der sich auch in einem Standphasendämpfungsniveau befindet, so dass bereits zu Beginn der Einbeugung ein ausreichend hoher Widerstand und Flexionsmoment anliegt, um dem Patienten ein sicheres Gefühl zu vermitteln. Der Anfangswert kann zwischen 40% und 70% des Maximalwiderstandes liegen.

Die Widerstandseinheit kann beispielsweise als Aktuator, beispielsweise als hydraulische, pneumatische, magnetorheologische, magnetische, elektrische, mechanische oder elektromagnetische Widerstandseinheit ausgestaltet sein. Bei hydraulischen oder pneumatischen Widerstandseinheiten werden Überströmkanäle geschlossen, so dass diese Überströmkanäle kein Medium mehr aus einer Extensionskammer in eine Flexionskammer strömen kann. Auf diese Weise kann der Fluss des Mediums zwischen der Extensionskammer und der Flexionskammer ggf. auch vollständig unterbunden werden. Bei mechanischen Widerstandseinrichtungen wird beispielsweise die Reibung soweit erhöht, dass keine weitere Flexion stattfinden kann. Gleiches gilt für elektrisch aktuierte Widerstandseinheiten.

Es können auch Aktuatoren zum Einsatz kommen, die sowohl Energie aktiv in das System einbringen, als auch umgekehrt dem System Energie entziehen und auf diese Weise als Widerstandseinheit wirken. Aktuatoren können beispielsweise als Elektromotoren, hydraulische oder pneumatische Pumpen oder piezoelektrische Elemente ausgebildet sein.

Nachfolgend wird ein Ausführungsbeispiel der Erfindung anhand der beigefügten Figuren näher erläutert. Es zeigen:
- Figur 1-: eine schematische Darstellung einer Prothese;
- Figur 2 -: schematische Darstellung der Dämpferanordnung an dem künstlichen Gelenk sowie
- Figur 3 -: schematische Darstellung der Widerstandseinheit zwischen Oberteil und Unterteil.

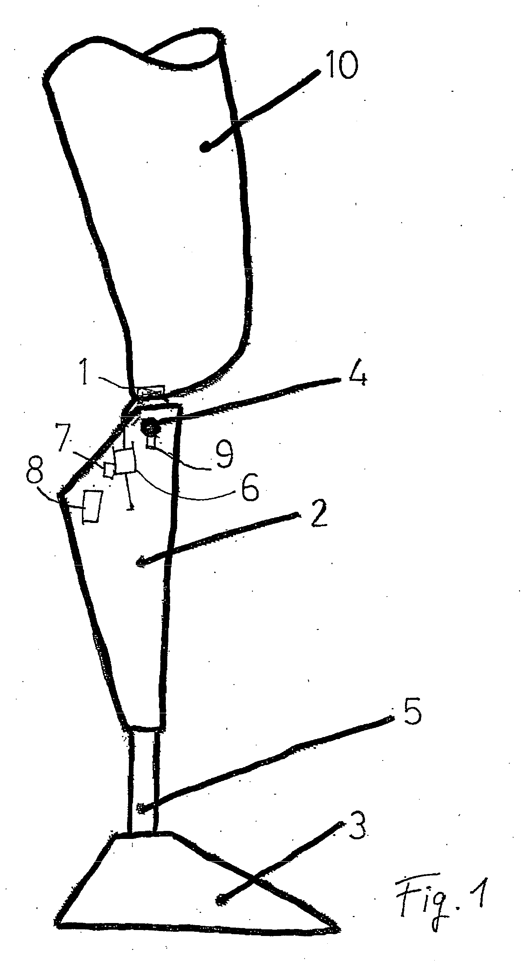

In der Figur 1 ist in einer schematischen Darstellung eine Beinprothese mit einem Oberteil 1 dargestellt, an dem ein Oberschenkelschaft 10 zur Aufnahme eines Oberschenkelstumpfes befestigt ist. An dem Oberteil 1 ist ein Unterteil 2 in Gestalt eines Unterschenkelteils schwenkbar angeordnet. Das Unterteil 2 ist an dem Oberteil 1 um eine Schwenkachse 4 schwenkbar gelagert. Das Unterteil 2 weist ein Unterschenkelrohr 5 auf, an dessen distalen Ende ein Prothesenfuß 3 befestigt ist, in dem eine Einrichtung zur Ermittlung der wirksamen Axialkraft auf das Unterschenkelrohr 5 sowie des Knöchelmomentes, das um die Befestigungsstelle des Prothesenfußes 3 an dem Unterschenkelrohr 5 wirksam ist, untergebracht sein kann.

In oder an dem Unterteil 2 ist eine Widerstandseinrichtung 6, die beispielsweise als Dämpfer oder Aktuator ausgebildet sein kann, angeordnet, die sich zwischen dem Oberteil 1 und dem Unterteil 2 abstützt, um einen einstellbaren Extensionswiderstand und Flexionswiderstand bereitzustellen. Der Widerstandseinrichtung 6 ist eine Verstelleinrichtung 7 zugeordnet, beispielsweise ein Motor, ein Magnet oder ein anderer Aktuator, über den der jeweilige Widerstand R innerhalb der Widerstandseinrichtung 6 verändert werden kann. Ist die Widerstandseinrichtung 6 als Hydraulikdämpfer oder Pneumatikdämpfer ausgebildet, kann über die Verstelleinrichtung 7 der jeweilige Strömungsquerschnitt eines Überströmkanals vergrößert oder verkleinert werden. Ebenso kann durch die Verstelleinrichtung 7 der Strömungswiderstand auf eine andere Art und Weise variiert werden. Dies kann beispielsweise durch Öffnen oder Schließen von Ventilen oder Veränderungen von Viskositäten oder magnetorheologischer Eigenschaften geschehen. Ist die Widerstandseinrichtung als ein Elektromotor im Generatorbetrieb ausgebildet, kann durch Veränderung des elektrischen Widerstandes eine Vergrößerung oder Verringerung der jeweiligen Widerstände gegen eine Flexion oder Extension eingestellt werden.

Um die Verstelleinrichtung 7 aktivieren oder deaktivieren zu können, ist eine Steuerungseinrichtung 8 dem Unterteil 2 zugeordnet, insbesondere in einer Unterschenkelverkleidung angebracht, über die ein entsprechendes Aktivierungs- oder Deaktivierungssignal an die Verstelleinrichtung 7 abgegeben wird. Die Verstelleinrichtung 7 wird auf der Basis von Sensordaten aktiviert oder deaktiviert, die Sensordaten werden von einem oder mehreren Sensoren 9 geliefert, die an dem künstlichen Kniegelenk angeordnet sind. Dies können Winkelsensoren, Beschleunigungssensoren und/oder Kraftsensoren sein. Die Sensoren 9 sind mit der Steuereinrichtung 8 verbunden, beispielsweise per Kabel oder über eine drahtlose Sendeeinrichtung. In dem dargestellten Ausführungsbeispiel ist der Sensor 9 unter anderem als Kniewinkelsensor ausgebildet.

Über die Sensoren 9 wird der gesamte Schrittzyklus von dem Fersenstoß (Heel Strike) bis zum erneuten, nachfolgenden Heel Strike HS überwacht, somit auch die gesamte Schwungphase mit der Schwungphasenextension und der Schwungphasenflexion.

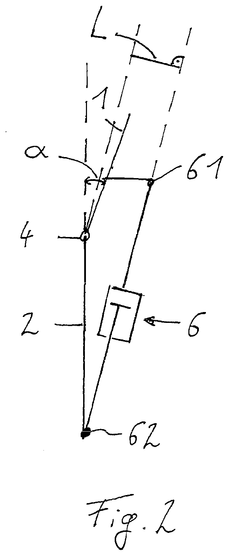

In der Figur 2 ist in einer schematischen Darstellung das künstliche Kniegelenk mit dem Oberteil 1, dem Unterteil 2, der Schwenkachse 4 und der dazwischen angeordneten Widerstandseinheit 6 dargestellt. Die Widerstandseinheit 6 ist als linear wirkende Widerstandseinheit dargestellt. Die Widerstandseinheit 6 stellt einen Widerstand R in Gestalt einer Widerstandskraft gegen die Einbeugung des künstlichen Kniegelenks um die Schwenkachse 4 bereit. Die Widerstandseinheit 6 ist an einem oberen Anlenkpunkt 61 an dem Oberteil 1 und an einem unteren Anlenkpunkt 62 an dem Unterteil 2 festgelegt. Im darstellten Ausführungsbeispiel ist die Widerstandseinheit 6 ein hydraulischer oder pneumatischer Dämpfer, grundsätzlich sind jedoch auch andere linearwirkende Widerstandseinheiten oder Einrichtungen, die einen Widerstand R gegen eine Verlagerung zweier Bauteile zueinander bereitstellen, geeignet, als Widerstandseinheit eingesetzt zu werden. Durch eine Verbindungslinie zwischen dem oberen Anlenkpunkt 61 und dem unteren Anlenkpunkt 62 verläuft die Kraftwirkungslinie der Widerstandseinheit.

In der Figur 2 ist der Abstand L der Wirkungslinie von der Schwenkachse 6 dargestellt. Der Abstand L ergibt sich aus dem Abstand der Wirkungslinie zwischen dem oberen Anlenkpunkt 61 und dem unteren Anlenkpunkt 62 der Widerstandseinheit 6 und einer gedachten Parallelen dazu, die durch die Schwenkachse 4 geht. In einer maximal extendierten Stellung, in der Streckstellung des künstlichen Kniegelenkes, stehen das Oberteil 1 und das Unterteil 2 im Wesentlichen senkrecht aufeinander, der Kniewinkel α ist 0°. Um überhaupt eine Dämpfung bewirken zu können, muss auch in der maximal extendierten Stellung ein Abstand L zwischen der Wirkungslinie der Widerstandseinheit 6 und der Schwenkachse 4 vorhanden sein. Üblicherweise weist das Kniegelenk einen Schwenkbereich auf, der größer als 100° ist, um ein bequemes Sitzen und gegebenenfalls auch ein Hinknien zu ermöglichen. Würde in der maximal gestreckten Stellung des künstlichen Knies ein maximaler Abstand anliegen, wäre nach Einbeugung des künstlichen Knies um 90° eine Totpunktlage erreicht. Das heißt, dass bei einem linear wirkenden Dämpfer eine Bewegungsumkehr stattfinden würde, im Bereich des Totpunktes wäre der Abstand Null, somit wäre auch das maximal aufbringbare Widerstandsmoment Null, da sich das Widerstandsmoment gegen eine Flexion aus dem Produkt der Widerstandskraft der Widerstandseinheit 6 mit dem Abstand L als Hebelarm ergibt.

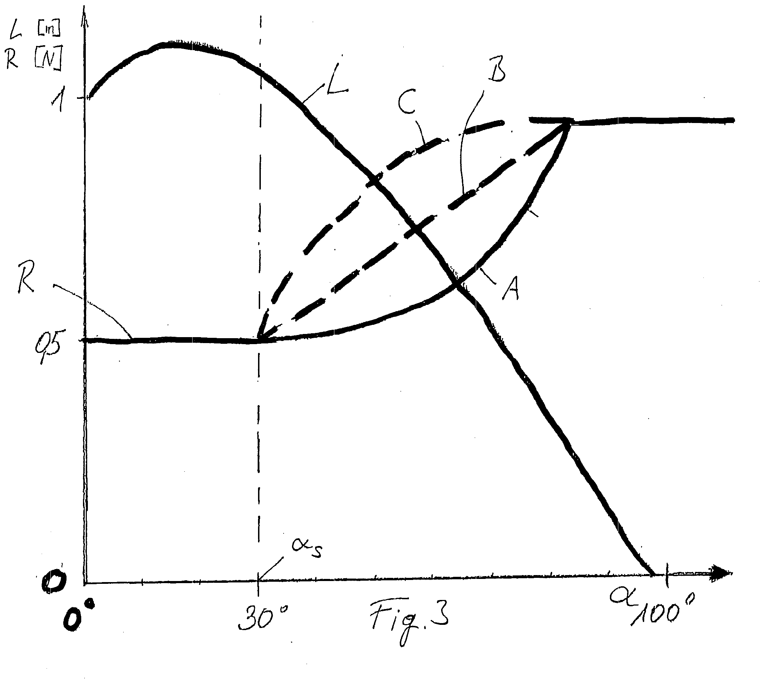

Figur 3 zeigt über den Kniewinkel α aufgetragenen Verlauf eines normierten Hebelarmwertes, wobei der Hebelarm L bei einem Kniewinkel α von 0°, also bei einer maximal extendierten Stellung, als 1 angenommen wird. Ebenfalls eingetragen ist der Verlauf des Widerstandes R der Widerstandseinheit 6 über den Kniewinkel α.

Der Verlauf des Hebelarmes L steigt zunächst an, bis der Abstand von der Schwenkachse 4 maximal wird. Dies ist im dargestellten Ausführungsbeispiel bei einem Kniewinkel α von ungefähr 15° der Fall. Anschließend fällt aufgrund der angenähert kreisförmigen Bewegung des oberen Anlenkpunktes 6 um die Schwenkachse 4 der Wert des Abstandes L ab, bis bei einem Kniewinkel von circa 100° die Verbindungslinie zwischen dem oberen Anlenkpunkt 61 und dem unteren Anlenkpunkt 62 durch die Schwenkachse 4 verläuft. Wird das Kniegelenk weiter eingebeugt, führt dies zu einer Bewegungsumkehr innerhalb der Widerstandseinheit 6, beispielsweise zu einer Umkehr eines Hydraulik- oder Pneumatikkolbens, eines mechanischen Widerstandes, beispielsweise eine Reibbremse oder eines elektrisch arbeitenden Aktuators.

Würde der Dämpferwiderstand R konstant bleiben, würde über einen zunehmenden Schwenkwinkel α ab Erreichen der Maximallänge des wirksamen Hebelarms L nur ein abnehmendes Widerstandsmoment bereitgestellt werden können, da sich der wirksame Hebel verringert. Erfindungsgemäß ist daher vorgesehen, dass in Abhängigkeit von dem Kniewinkel α der Dämpferwiderstand ab Erreichen eines Schwellwertes αₛ vergrößert wird, beispielsweise indem ein zunächst halb geschlossenes Ventil bei einer hydraulischen oder pneumatischen Widerstandseinheit 6 zunehmend geschlossen wird. In dem dargestellten Ausführungsbeispiel ist eine vollständige Sperrung des Ventils nicht vorgesehen, die bei einem Wert 1 vorliegen würde.

In der Darstellung der Figur 3 sind drei verschiedene Kurven der Widerstandsveränderung gezeigt. Die erste Kurve A mit einem progressiven Verlauf bis zu einem Kniewinkel von 90° bewirkt, dass ein maximales Widerstandsmoment ungefähr im Bereich einer Kniewinkelstellung zwischen 70° und 90° anliegt.

Der Kurvenverlauf B zeigt einen linearen Anstieg des Dämpfungswiderstandes, wodurch zunächst ein sich steigerndes Widerstandsmoment aufgrund einer vergleichsweise langsamen Verringerung der Hebellänge L einstellt, im weiteren Verlauf der Kniebeugung verringert sich dann das Widerstandsmoment.

Die Kennlinie C bewirkt ein zunächst ansteigendes Widerstandsmoment, das nach Erreichen eines Maximalwertes bei ungefähr 45° aufgrund der degressiven Kurve zunehmend verringert wird.

Das Steuerverfahren dient zur Anpassung des Widerstandsmomentes an die Bedürfnisse beim Niedersetzen. Um zu erkennen, ob ein Patient eine Hinsetzfunktion wünscht bzw. das Hinsetzen ausführt, kann entweder ein entsprechendes Steuerungsprogramm manuell ausgewählt oder aber über verschiedene Sensoren automatisch eine Unterscheidung auf der Grundlage der Auswertung von Sensordaten vorgenommen werden. Hinzu können Beschleunigungssensoren, Lagesensoren, Kraftsensoren und Absolutwinkelsensoren verwendet werden, die eine Unterscheidung zwischen Hinsetzen, Gehen in der Ebene, Bergabgehen, Gehen auf schiefen Ebenen oder Treppensteigen ermöglichen.

Mit dem erfindungsgemäßen Verfahren ist es möglich, die Mechanik des künstlichen Kniegelenkes unangetastet zu lassen, lediglich der Dämpferwiderstand R wird erhöht, beispielsweise durch zunehmendes Sperren eines Flüssigkeitsdurchganges, durch Erhöhen eines Reibwertes oder durch Erhöhen eines elektrischen Widerstandes. Dies bewirkt, dass bei einem sich vergrößernden Kniewinkel das Widerstandsmoment gegen eine Einbeugung hoch gehalten wird.

## Patentansprüche

1. Verfahren zur Steuerung einer Dämpfungsveränderung bei einem künstlichen Kniegelenk einer Orthese oder Prothese, wobei das künstliche Kniegelenk ein Oberteil (1), ein um eine Schwenkachse (4) schwenkbar daran gelagertes Unterteil (2) und eine Widerstandseinheit (6) aufweist, die an dem Oberteil (1) an einem oberen Anlenkpunkt (61) und an dem Unterteil (2) an einem unteren Anlenkpunkt (62) befestigt ist, um einen Widerstand gegen eine Einbeugung oder Streckung des künstlichen Kniegelenks bereitzustellen, wobei der Widerstandseinheit (6) eine Verstelleinrichtung (7) zur Veränderung des Flexionswiderstandes zugeordnet ist, **dadurch gekennzeichnet, dass** der Verstelleinrichtung (7) eine mit Sensoren (9) verbundene Steuerungseinrichtung (8) zugeordnet ist und automatisch auf der Grundlage einer Auswertung von Sensordaten und/oder durch eine manuelle Auswahl eine Hinsetzfunktion aktiviert wird, in der der Widerstand (R) der Widerstandseinheit (6) ab einem Kniewinkelschwellwert mit zunehmendem Kniewinkel (α) vergrößert wird um eine Anpassung des Widerstandsmomentes an die Bedürfnisse beim Niedersetzen zu erzielen.

2. Verfahren nach Anspruch 1, **dadurch gekennzeichnet, dass** der Widerstand (R) innerhalb der Widerstandseinheit (6) mit zunehmendem Kniewinkel (α) linear, progressiv oder degressiv erhöht wird.

3. Verfahren nach Anspruch 1 oder 2, **dadurch gekennzeichnet, dass** als Kniewinkelschwellwert (αₛ), ab dem eine Veränderung des Widerstandes (R) der Widerstandseinheit (6) erhöht wird, derjenige Kniewinkel (α) festgelegt wird, bei dem der Abstand (L) der Verbindungslinie zwischen dem oberen Anlenkpunkt (61) und dem unteren Anlenkpunkt (62) zu der Schwenkachse (4) maximal ist

4. Verfahren nach einem der voranstehenden Ansprüche, **dadurch gekennzeichnet, dass** die Widerstandseinheit (6) einen hydraulischen oder pneumatischen Dämpfer mit zumindest einem Überströmkanal und einer verstellbaren Drossel aufweist.

5. Verfahren nach einem der voranstehenden Ansprüche, **dadurch gekennzeichnet, dass** Widerstandseinheit (6) einen mechanischen Widerstand aufweist.

6. Verfahren nach einem der voranstehenden Ansprüche, **dadurch gekennzeichnet, dass** die Widerstandseinheit (6) einen elektrisch betriebenen Aktuator aufweist.

7. Verfahren nach einem der voranstehenden Ansprüche, **dadurch gekennzeichnet, dass** der Widerstand (R) in der Widerstandseinheit (6) dergestalt erhöht, dass das Flexionsmoment in einem Kniewinkelbereich zwischen 70° und 90° maximal wird.

8. Verfahren nach einem der voranstehenden Ansprüche, **dadurch gekennzeichnet, dass** ab einem Kniewinkelschwellwert (αₛ) von 30° der Widerstand (R) der Widerstandseinheit (6) erhöht wird.

9. Verfahren nach einem der voranstehenden Ansprüche, **dadurch gekennzeichnet, dass** der Widerstand (R) in der Widerstandseinheit (6) umgekehrt proportional zum Verlauf der Hebelarmveränderung während des Einbeugens verändert wird, um ein konstantes oder nahezu konstantes Flexionsmoment bereitzustellen.

10. Verfahren nach einem der voranstehenden Ansprüche, **dadurch gekennzeichnet, dass** der Anfangswiderstand (R₀) auf einem Niveau eingestellt wird, das einem Standphasendämpfungsniveau entspricht.

11. Verfahren nach einem der voranstehenden Ansprüche, **dadurch gekennzeichnet, dass** als Sensoren (9) Beschleunigungssensoren, Lagesensoren, Kraftsensoren und Absolutwinkelsensoren verwendet werden, die eine Unterscheidung zwischen Hinsetzen, Gehen in der Ebene, Bergabgehen, Gehen auf schiefen Ebenen oder Treppensteigen ermöglichen.

## Claims

1. A method for controlling a change in damping in an artificial knee joint of an orthosis or prosthesis, wherein the artificial knee joint has an upper part (1), a lower part (2) which is mounted on said upper part pivotably about a pivot axis (4), and a resistance unit (6) which is fastened to the upper part (1) at an upper articulation point (61) and to the lower part (2) at a lower articulation point (62) for the purposes of providing a resistance to a flexion or extension of the artificial knee joint, wherein the resistance unit (6) is assigned an adjustment device (7) for changing the flexion resistance, **characterized in that** a control device (8) connected to sensors (9) is associated with the adjusting device (7) and a sit-down function is automatically activated on the basis of an evaluation of sensor data and/or by manual selection, in the sit-down function the resistance (R) of the resistance unit (6) is increased from a knee angle threshold value with increasing knee angle (α) in order to achieve an adaptation of the resistance torque to the needs during sitting down.

2. The method as claimed in claim 1, **characterized in that** the resistance (R) within the resistance unit (6) is increased linearly, progressively or degressively with increasing knee angle (α).

3. The method as claimed in claim 1 or 2, **characterized in that** that knee angle (α) at which the spacing (L) of the connecting line between the upper articulation point (61) and the lower articulation point (62) to the pivot axis (4) is at a maximum is set as a knee angle threshold value (αₛ), above which a change in the resistance (R) of the resistance unit (6) is increased.

4. The method as claimed in one of the preceding claims, **characterized in that** the resistance unit (6) has a hydraulic or pneumatic damper with at least one flow transfer channel and with an adjustable throttle.

5. The method as claimed in one of the preceding claims, **characterized in that** the resistance unit (6) has a mechanical resistance.

6. The method as claimed in one of the preceding claims, **characterized in that** the resistance unit (6) has an electrically operated actuator.

7. The method as claimed in one of the preceding claims, **characterized in that** the resistance (R) in the resistance unit (6) increases such that the flexion moment is at a maximum in a knee angle range between 70° and 90°.

8. The method as claimed in one of the preceding claims, **characterized in that**, above a knee angle threshold value (αₛ) of 30°, the resistance (R) of the resistance unit (6) is increased.

9. The method as claimed in one of the preceding claims, **characterized in that** the resistance (R) in the resistance unit (6) is changed in a manner inversely proportional to the profile of the lever arm change during the flexion, so as to provide a constant or virtually constant flexion moment.

10. The method as claimed in one of the preceding claims, **characterized in that** the initial resistance (R₀) is set to a level which corresponds to a stance phase damping level.

11. Method according to one of the preceding claims, **characterized in, that** acceleration sensors, position sensors, force sensors and absolute angle sensors are used as sensors (9), which enable a distinction to be made between sitting down, walking on level ground, walking downhill, walking on inclined planes or climbing stairs.

## Revendications

1. Procédé de commande d'une modification de l'amortissement dans une articulation de genou artificielle d'une orthèse ou d'une prothèse, l'articulation de genou artificielle comprenant une partie supérieure (1), une partie inférieure (2) montée sur celle-ci de manière à pouvoir pivoter autour d'un axe de pivotement (4), et une unité de résistance (6) qui est fixée à la partie supérieure (1) en un point d'articulation supérieur (61) et à la partie inférieure (2) en un point d'articulation inférieur (62), afin de fournir une résistance à la flexion ou à l'extension de l'articulation de genou artificielle, un dispositif de réglage (7) étant associé à l'unité de résistance (6) pour modifier la résistance à la flexion,
**caractérisé en ce qu'**un dispositif de commande (8) relié à des capteurs (9) est associé au dispositif de réglage (7), et une fonction de s'asseoir est activée automatiquement sur la base d'une évaluation de données de capteurs et/ou par une sélection manuelle, fonction dans laquelle la résistance (R) de l'unité de résistance (6) est augmentée à partir d'une valeur seuil d'angle de genou au fur et à mesure que l'angle de genou (α) s'accroît, afin d'obtenir une adaptation du couple de résistance selon les besoins, lors de l'opération de s'asseoir.

2. Procédé selon la revendication 1,
**caractérisé en ce que** la résistance (R) au sein de l'unité de résistance (6) est augmentée de manière linéaire, progressive ou dégressive au fur et à mesure que l'angle de genou (α) s'accroît.

3. Procédé selon la revendication 1 ou 2,
**caractérisé en ce que** l'on fixe comme valeur seuil d'angle de genou (αₛ) à partir de laquelle une modification de la résistance (R) de l'unité de résistance (6) est augmentée, l'angle de genou (α) pour lequel la distance (L) de la ligne de liaison entre le point d'articulation supérieur (61) et le point d'articulation inférieur (62) par rapport à l'axe de pivotement (4) est maximale.

4. Procédé selon l'une des revendications précédentes,
**caractérisé en ce que** l'unité de résistance (6) comprend un amortisseur hydraulique ou pneumatique présentant au moins un canal de trop-plein et un étranglement réglable.

5. Procédé selon l'une des revendications précédentes,
**caractérisé en ce que** l'unité de résistance (6) présente une résistance mécanique.

6. Procédé selon l'une des revendications précédentes,
**caractérisé en ce que** l'unité de résistance (6) comprend un actionneur à commande électrique.

7. Procédé selon l'une des revendications précédentes,
**caractérisé en ce que** la résistance (R) dans l'unité de résistance (6) est augmentée de telle sorte que le couple de flexion devient maximal dans une plage d'angle de genou entre 70° et 90°.

8. Procédé selon l'une des revendications précédentes,
**caractérisé en ce qu'**à partir d'une valeur seuil d'angle de genou (αₛ) de 30°, la résistance (R) de l'unité de résistance (6) est augmentée.

9. Procédé selon l'une des revendications précédentes,
**caractérisé en ce que** la résistance (R) dans l'unité de résistance (6) est modifiée de manière inversement proportionnelle à l'évolution de la variation du bras de levier pendant la flexion, afin de fournir un couple de flexion constant ou quasi-constant.

10. Procédé selon l'une des revendications précédentes,
**caractérisé en ce que** la résistance initiale (R₀) est réglée à un niveau qui correspond au niveau d'amortissement de la phase debout.

11. Procédé selon l'une des revendications précédentes,
**caractérisé en ce que** l'on utilise comme capteurs (9) des capteurs d'accélération, des capteurs de position, des capteurs de force et des capteurs d'angle absolu qui permettent de faire la distinction entre les opérations de s'asseoir, de marcher sur le plat, de descendre une pente, de marcher sur un plan incliné ou de monter ou descendre un escalier.
